# EUROPEAN PATENT SPECIFICATION

(11) **EP 3 859 008 B1**
(45) Date of publication and mention of the grant of the patent: **16.11.2022**
(21) Application number: 19878222.9
(22) Date of filing: 05.09.2019
(51) Int. Cl.: C12Q 1/06, C12M 1/00, C12M 1/34, C12M 1/36

(54) **METHOD FOR ESTIMATING CELL COUNT, AND DEVICE FOR ESTIMATING CELL COUNT**
VERFAHREN ZUR BESTIMMUNG DER ZELLZAHL UND VORRICHTUNG ZUR BESTIMMUNG DER ZELLZAHL
PROCÉDÉ D'ESTIMATION DE NOMBRE DE CELLULES, ET DISPOSITIF D'ESTIMATION DE NOMBRE DE CELLULES

(30) Priority: 02.11.2018 JP 2018207092
(43) Date of publication of application: 04.08.2021
(73) Proprietor: PHC Holdings Corporation, Tokyo 105-8433 (JP)
(72) Inventor: SAKURAI, Tsubasa, Toon-shi, Ehime 791-0395 (JP); TAIRA, Keisuke, Toon-shi, Ehime 791-0395 (JP)
(74) Representative: Grünecker Patent- und Rechtsanwälte PartG mbB
(86) International application number: PCT/JP2019/034982
(87) International publication number: WO 2020/090225

(56) References cited:
- JP-A- 2010 081 809
- JP-A- 2012 520 078
- US-A- 4 592 994
- US-A1- 2009 104 594

## Description

### [Technical field]

The present disclosure relates to a method and device for estimating the number of cells.

### [Background art]

Examples of a method for treating cancer and leukemia include, for example, a method for removing cells involved in a patient's immunity from the patient's body, artificially culturing the cells outside the patient's body, and then returning them to the patient's body. In such a case, there is a demand, in production thereof, to estimate the number of cells in culture non-invasively and continuously. In the present specification, the term "non-invasively" refers to a manner without involving sampling target cells.

It is known that there is a correlation between concentration of cellular metabolite and the number of cells. Thus, as a method for estimating the number of cells in culture, considered is a method for estimating the cellular metabolite concentration that changes over time as cells are cultured in culture media and comparing the estimated concentration with an existing database (see, for example, Patent Literature 1). The database stores information regarding the relationship between cellular metabolite concentration and the number of cells. In the present specification, the "cellular metabolite" is defined as a cell-consumed substance and a cell-produced substance to be used in cellular metabolism. Such cell-consumed substances are consumed in the process of cellular metabolism. Accordingly, they decrease over time as cells are cultured. Examples of cell-consumed substances include glucose. Such cell-produced substances are produced in the process of cellular metabolism. Accordingly, they increase over time as cells are cultured. Examples of cell-produced substances include lactate.

Information regarding the relationship between cellular metabolite concentration and the number of cells is a prerequisite for estimation of the number of cells, and thus needs to have a high degree of accuracy. Examples of a method for obtaining information regarding the relationship between cellular metabolite concentration and the number of cells with high accuracy includes a bioassay. A specific example thereof is as follows: collecting some of the cell media in which cells are being cultured; adding a reagent that reacts only with a specific cellular metabolite to the collected media; and detecting a luminescence signal produced by reacting the reagent with the cellular metabolite in the media. The luminescence signal may be of any type, such as visible light, fluorescence, and phosphorescence, as long as it is detectable.

Here, the intensity of the luminescence signal is proportional to the cellular metabolite concentration. Accordingly, by detecting the luminescence signal, the concentration of the cellular metabolite contained in the collected media can be measured. The number of cells in the media can be measured by a known method such as a cell counter. This makes it possible to obtain information about the number of cells at a certain cellular metabolite concentration. Similarly, by obtaining information about the number of cells at different cellular metabolite concentrations, it is possible to obtain information regarding the relationship between cellular metabolite concentration and the number of cells. Alternatively, a color reagent and the like that exhibit coloration depending on the cellular metabolite concentration may be used as a reagent.

The bioassay, however, is an invasive method, and thus it is inevitable that a sampling device or the like contacts culture media. Accordingly, there is such an issue that the media might be contaminated, for example. In addition, since it is difficult to perform sampling many times during culture, information about the number of cells can be obtained only intermittently.

Thus, apart from the bioassay, spectroscopic approaches can be considered. Specifically, a culture medium is irradiated with light having a wavelength that does not adversely affect cells, and the absorbance and/or the degree of scattering thereof are measured. The values thus obtained are subject to an analytical method such as multivariate analysis, thereby being able to estimate cellular metabolite concentration. The cellular metabolite concentration estimated in this way is compared with the relationship information obtained from the bioassay, thereby being able to estimate the number of cells in the estimated cellular metabolite concentration. Spectroscopy has an advantage in being able to estimate the cellular metabolite concentration non-invasively and continuously.

[PTL 1] Japanese Patent Application Publication No. 2014-45663

US 2009/104594 A1 relates to a bioreactor including a sensor providing measurements of a quantity that correlates with a desired product quality attribute.

US 4 592 994 A relates to a method wherein a number of microorganism cells in in a sample is estimated based on the quantity of products generated by cell metabolism.

However, in general, cellular metabolite concentration can be accurately estimated by spectroscopy only when the cellular metabolite concentration is high. Using examples of glucose and lactate, glucose concentration decreases as cells are cultured, and thus cannot be estimated accurately after a certain culturing period has elapsed. In contrast, lactate concentration increases as cells are cultured, and thus can be accurately estimated only after a certain culturing period has elapsed.

The present disclosure is directed to provision of a method and device for estimating the number of cells non-invasively and continuously.

The invention is defined by the independent claims. The dependent claims describe advantageous embodiments.

An aspect of the present disclosure is a method for estimating the number of cells, the method comprising: estimating a concentration of a first cellular metabolite contained in a culture medium in which certain cells are cultured, using a method capable of obtaining a value corresponding to a concentration of a cellular metabolite contained in a culture medium; estimating, when the estimated concentration of the first cellular metabolite is equal to or higher than a threshold, the number of the certain cells by applying the estimated concentration of the first cellular metabolite to previously obtained first relationship information indicating a relationship between a concentration of the first cellular metabolite and the number of the certain cells; estimating, when the estimated concentration of the first cellular metabolite is lower than the threshold, a concentration of a second cellular metabolite contained in the culture medium, using the method; and estimating, when the estimated concentration of the second cellular metabolite is equal to or higher than a threshold, the number of the certain cells by applying the estimated concentration of the second cellular metabolite to previously obtained second relationship information indicating a relationship between a concentration of the second cellular metabolite and the number of the certain cells.

In addition, another aspect of the present disclosure is a device that estimates the number of cells, the device comprising: a first estimation unit that estimates a concentration of a cellular metabolite contained in a culture medium in which certain cells are cultured, based on data measured using a method capable of obtaining a value corresponding to a concentration of a cellular metabolite contained in a culture medium; a determination unit that determines whether the estimated concentration of the cellular metabolite is equal to or higher than a threshold; and a second estimation unit that estimates the number of certain cells, by applying the estimated concentration of the cellular metabolite to previously obtained relationship information indicating a relationship between a concentration of the cellular metabolite and the number of the certain cells, according to a determination result obtained by the determination unit, the second estimation unit being configured to, when a concentration of a first cellular metabolite estimated by the first estimation unit is determined to be equal to or higher than the threshold, estimate the number of the certain cells by applying the estimated concentration of the first cellular metabolite to previously obtained first relationship information indicating a relationship between a concentration of the first cellular metabolite and the number of the certain cells, the first estimation unit being configured to, when the estimated concentration of the first cellular metabolite is determined to be lower than the threshold, estimate a concentration of a second cellular metabolite contained in the culture medium; and the second estimation unit being configured to, when the estimated concentration of the second cellular metabolite is determined to be equal to or higher than the threshold, estimate the number of the certain cells by applying the estimated concentration of the second cellular metabolite to previously obtained second relationship information indicating a relationship between a concentration of the second cellular metabolite and the number of the certain cells.

According to the present disclosure, the number of cells can be estimated non-invasively and continuously.

### [Brief description of the drawings]

Fig. 1 is a diagram illustrating a device for estimating the number of cells according to a first example.
Fig. 2 is a diagram illustrating a control unit according to a first example.
Fig. 3 is a flowchart of a method for estimating the number of cells according to a first example.
Fig. 4 is a diagram illustrating a device for estimating the number of cells according to a second example.
Fig. 5 is a diagram illustrating a control unit according to a second example.
Fig. 6 is a flowchart of a method for estimating the number of cells according to a second example.
Fig. 7 is a graph illustrating a relationship between the number of cells and the number of days in culture.
Fig. 8A is a graph illustrating a relationship between glucose concentration and the number of days in culture.
Fig. 8B is a graph illustrating a relationship between lactate concentration and the number of days in culture.
Fig. 9A is a graph illustrating a relationship between glucose concentration estimated using multivariate analysis and concentration obtained using a bioassay.
Fig. 9B is a graph illustrating a relationship between lactate concentration estimated using multivariate analysis and concentration obtained using a bioassay.

### [Description of Embodiments]

Although an embodiment of the present disclosure is described below, the scope of the present disclosure is not limited to the description including examples.

### <Acquisition of relationship information>

Relationship information indicates a relationship between cellular metabolite concentration and the number of certain cells. The relationship information is provided for each cellular metabolite.

Relationship information can be obtained by various methods. A bioassay is particularly preferable since it is capable of obtaining highly accurate relationship information. The bioassay is not limited to a method of using a reagent as described above, but various methods such as mass spectrometry, antibody method, and the like are usable as long as they are methods for acquiring and analyzing a biological response.

### <Method for obtaining value corresponding to concentration of cellular metabolite contained in culture media>

It is preferable that the method is capable of obtaining measurement results non-invasively and continuously. Examples of a value corresponding to the concentration of cellular metabolite contained in culture media include optical and electrical values. These values can be obtained using a known sensor, for example.

Examples of the method include such spectroscopy as described above. The spectroscopy is a method for measuring the absorbance and/or the degree of scattering through irradiating the culture media with light having a wavelength that does not adversely affect cells. In addition, it is a method capable of obtaining measurement results non-invasively and continuously. The measurement results thus obtained is subject to an analytical method such as multivariate analysis, thereby being able to estimate the cellular metabolite concentration (described later).

The spectroscopy includes near-infrared spectroscopy, visible spectroscopy, fluorescence spectroscopy, Raman spectroscopy, and the like that are usable depending on the cellular metabolite whose concentration is to be estimated. Fluorescence spectroscopy is a method for measuring fluorescence intensity through adding, to the culture media, a probe that emits fluorescence in response to specifically binding to a cellular metabolite. In the case of using fluorescence spectroscopy, it is preferable to immobilize the probe to a substrate that functions as a sensor and cover the probe with a protective film. Covering the probe with a protective film prevents the probe from leaking out and enables non-invasive measurements. The spectroscopy is not limited thereto, as long as it do not adversely affect cells.

An electrochemical system serves as an alternative to the spectroscopy. The electrochemical system is a method for obtaining an electrical value using an enzyme that acts on an analyte as the substrate. The electrical value corresponds to the concentration of the analyte. The electrochemical system is a method capable of obtaining measurement results non-invasively and continuously. With a cellular metabolite used as the analyte, it is possible to obtain the electrical value corresponding to the cellular metabolite concentration.

As a sensor to be used for the electrochemical system, it is possible to employ an enzyme sensor capable of continuously measuring glucose or an enzyme sensor capable of continuously measuring lactate. Although these enzyme sensors are non-invasive sensors that do not require sampling of cells, they need to be grounded to the culture media during measurement. Thus, in the case of using an electrochemical system, it is preferable to provide a protective film on the ground surface of such an enzyme sensor. The protective film reduces the adverse effects on the cells caused by grounding the enzyme sensor. The sensor used in the electrochemical system is not limited to an enzyme sensor with a protective film, as long as it does not adversely affect the cells.

### <Estimation of cellular metabolite concentration>

Cellular metabolite concentration can be obtained by using a statistical method and analyzing values of the absorbance and/or the degree of scattering, or electrical values obtained by the above methods.

A known technique can be used as the statistical method, depending on the number of data measured by the methods mentioned above, the wavelength of light irradiation, and other conditions.

### == First Example ==

Here, the following is a first example in which the cellular metabolite concentration is estimated using spectroscopy as a method of obtaining a value corresponding to concentration of the cellular metabolite contained in culture media and using multivariate analysis as the statistical method.

### (Estimation of the number of cells)

The number of cells can be estimated by applying the estimated cellular metabolite concentration to the relationship information corresponding to the cellular metabolite.

On the other hand, in the case of using spectroscopy, the accurate concentration can be estimated only when the cellular metabolite concentration is high.

Here, cell-consumed substances among the cellular metabolites decrease over time as cells are cultured. On the other hand, cell-produced substances among cellular metabolites increase over time as cells are cultured.

In other words, the concentration of cell-consumed substances cannot be accurately estimated in the late stage of culture. In addition, the concentration of cell-produced substances cannot be accurately estimated in the early stage of culture.

Thus, by the method according to an embodiment of the present disclosure, the number of cells are estimated based on either cell-consumed substance concentration or cell-produced substance concentration.

Specifically, when the estimated cell-consumed substance concentration is equal to or higher than a threshold, the number of certain cells is estimated by applying the estimated cell-consumed substance concentration to the previously obtained relationship information indicating the relationship between cell-consumed substance concentration and the number of certain cells.

On the other hand, when the estimated cell-consumed substance concentration is lower than a threshold, the cell-produced substance concentration is estimated. When the estimated cell-produced substance concentration is equal to or higher than a threshold, the number of certain cells is estimated by applying the estimated cell-produced substance concentration to the previously obtained relationship information indicating the relationship between cell-produced substance concentration and the number of certain cells.

The threshold is the lower limit of the concentration of a cellular metabolite at which the actual concentration thereof can be accurately estimated using spectroscopy, in cellular metabolites. The threshold is obtained using, for example, a cross-validation method. Specifically, near-infrared spectroscopy is used to measure the absorbance of a culture medium with a given concentration (referred to as a "measured concentration") of a cellular metabolite, and then multivariate analysis is used to estimate the concentration of the cellular metabolite from the absorbance. This process is repeated multiple times to accumulate data on the relationship between a measured concentration and an estimated concentration of the cellular metabolite. Next, the cross-validation method is used to delete the data of low concentrations from the accumulated measured data, until the correlation coefficient between the measured concentration and the estimated concentration of the cellular metabolite becomes 0.7 or higher. The lowest concentration value of the measured data obtained when the correlation coefficient becomes 0.7 or higher is set to the threshold.

The cellular metabolite is preferably a combination of a cell-consumed substance and a cell-produced substance. As described above, the actual concentration of the cell-consumed substance can be accurately estimated in the early to middle stages of culture during which the concentration thereof is high. On the other hand, the actual concentration of the cell-produced substance can be accurately estimated in the middle to late stages of the culture during which the concentration thereof is high. Accordingly, it is possible to estimate the number of cells at any stage of culture using the cell-consumed substance concentration or the cell-produced substance concentration.

Examples of a combination of the cell-consumed substance and the cell-produced substance include a combination of glucose and lactate, a combination of glutamine and glutamate, and the like. For example, T cells consume glucose and produce lactate.

In the above description, the cell-consumed substance is an example of a "first cellular metabolite" and the cell-produced substance is an example of a "second cellular metabolite." The relationship information indicating the relationship between cell-consumed substance concentration and the number of certain cells is an example of a "first relationship information," and the relationship information indicating the relationship between cell-produced substance concentration and the number of certain cells is an example of a "second relationship information." Note that, in the case where the cell-produced substance concentration is compared with a threshold before the cell-consumed substance concentration is compared therewith, the cell-produced substance corresponds to the "first cellular metabolite" and the cell-consumed substance corresponds to the "second cellular metabolite."

### (Device that measures the number of cells)

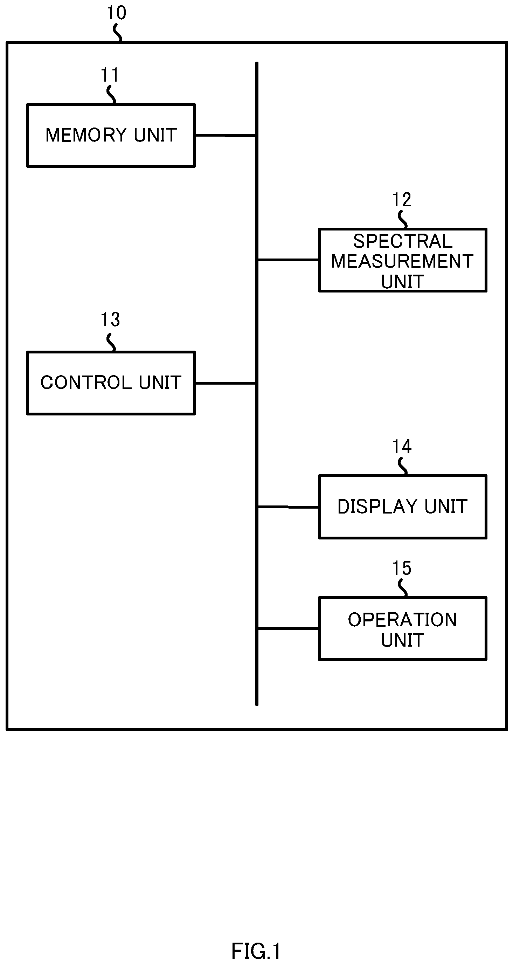

Referring to Fig. 1, a device 10 that estimates the number of cells according to a first example is described.

The device 10 comprises a memory unit 11, a spectral measurement unit 12, a control unit 13, a display unit 14, and an operation unit 15.

### [Memory unit]

The memory unit 11 stores various pieces of information about the device 10. The memory unit 11 according to an embodiment of the present disclosure stores previously obtained relationship information indicating the relationship between the concentrations of cellular metabolites and the number of cells.

### [Spectral measurement unit]

The spectral measurement unit 12 irradiates the culture media with light having a predetermined range of wavelengths, and receives absorbed light, reflected light and scattered light. The spectral measurement unit 12 outputs the received light to the control unit 13.

### [Control unit]

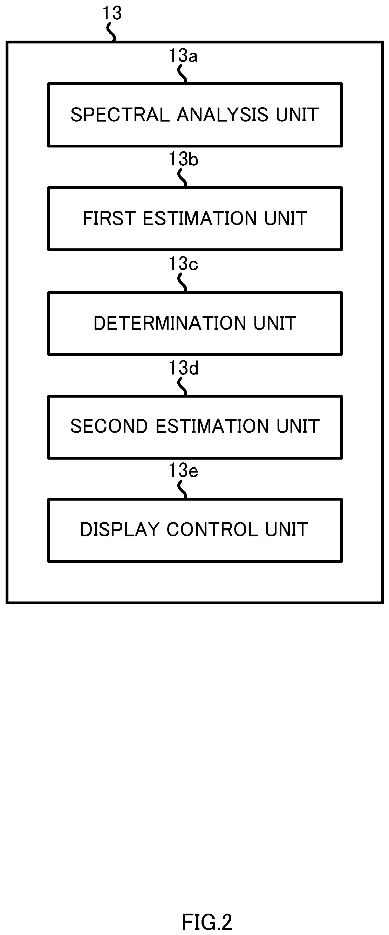

The control unit 13 comprises a CPU and a memory (not shown). The CPU implements various control functions by executing an operation program stored in the memory. The memory is a storage device to store programs to be executed by the CPU, temporarily store various pieces of information when a program is executed, and other. In an embodiment of the present disclosure, the control unit 13 functions as a spectral analysis unit 13a, a first estimation unit 13b, a determination unit 13c, a second estimation unit 13d, and a display control unit 13e (see Fig. 2).

The spectral analysis unit 13a measures the absorbance and/or the degree of scattering from the light received from the spectral measurement unit 12. A known method can be used to measure the absorbance and/or the degree of scattering.

The first estimation unit 13b estimates the concentration of a cellular metabolite contained in a culture medium in which certain cells are cultured, based on the data measured using spectroscopy. The data includes, for example, the absorbance obtained by the spectral analysis unit 13a, and the like. The method(s) described above can be used to estimate the concentration of the cellular metabolite.

The determination unit 13c determines whether the estimated concentration of the cellular metabolite is equal to or higher than a threshold.

The second estimation unit 13d estimates the number of certain cells by applying the estimated concentration of the cellular metabolite to the relationship information stored in the memory unit 11, according to the determination result obtained by the determination unit 13c.

Here, a specific process according to an embodiment of the present disclosure is described. First, the first estimation unit 13b estimates the concentration of the first cellular metabolite. The determination unit 13c determines whether the estimated concentration of the first cellular metabolite is equal to or higher than the threshold.

When it is determined that the estimated concentration of the first cellular metabolite is equal to or higher than the threshold, the second estimation unit 13d estimates the number of certain cells by applying the estimated concentration of the first cellular metabolite to the first relationship information stored in the memory unit 11.

On the other hand, when the estimated concentration of the first cellular metabolite is determined to be lower than the threshold, the first estimation unit 13b estimates the concentration of the second cellular metabolite contained in the culture medium. The determination unit 13c determines whether the estimated concentration of the second cellular metabolite is equal to or higher than a threshold.

When it is determined that the estimated concentration of the second cellular metabolite is equal to or higher than the threshold, the second estimation unit 13d estimates the number of certain cells by applying the estimated concentration of the second cellular metabolite to the second relationship information stored in the memory unit 11.

The display control unit 13e performs various display controls in the device 10. For example, the display control unit 13e displays the result of the estimation of the number of the cells on the display unit 14.

### [Display unit and operation unit]

The display unit 14, such as a display, is configured to display various information. The operation unit 15 is configured to input various instructions to the device 10, such as a mouse, a keyboard, a touch panel displayed on the display unit 14, and/or the like. The device 10 may also have a function of printing out the information displayed on the display unit 14 and/or outputting it from an external printer.

### (Method for estimating the number of cells)

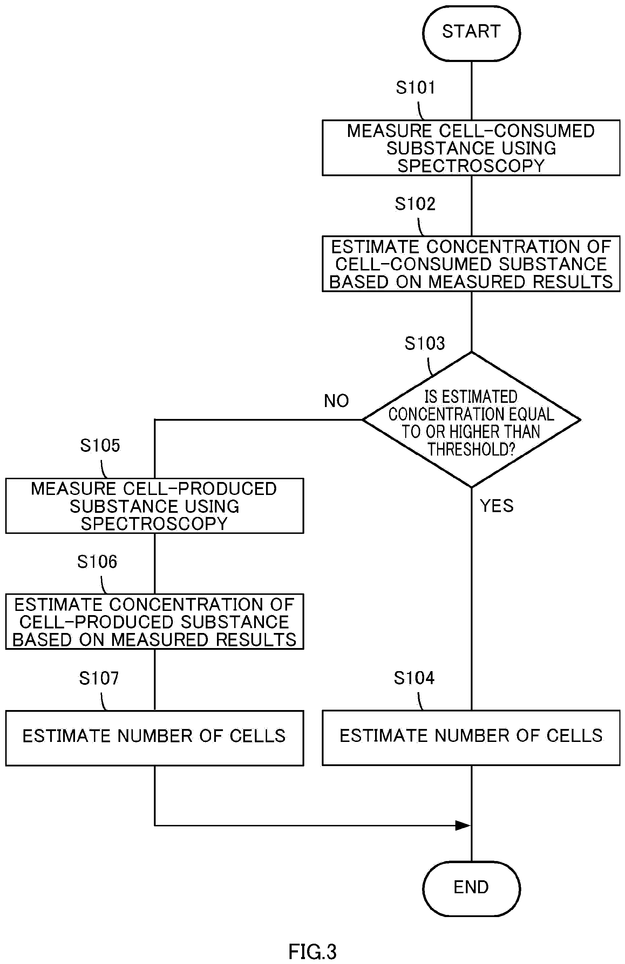

Next, referring to Fig. 3, described is a method for estimating the number of cells C using the device 10. Following is an example described assuming that cells C consume a cell-consumed substance X and produce a cell-produced substance Y. It is also assumed that the concentration of the cell-consumed substance X is estimated first. Furthermore, it is assumed that the first relationship information indicating the relationship between concentration of the cell-consumed substance X and the number of cells C, and the second relationship information indicating the relationship between concentration of cell-produced substance and the number of cells C are previously obtained using a bioassay.

First, a medium in which cells C are cultured is placed in the device 10.

The spectral measurement unit 12 irradiates the culture medium with light having a predetermined wavelength, receives absorbed light, and the like, and outputs the received light to the control unit 13.

The spectral analysis unit 13a measures the absorbance and the degree of scattering of the cell-consumed substance X contained in the culture medium in which the cells C are cultured (S101).

The first estimation unit 13b analyzes the data of the absorbance and the degree of scattering measured in S101 to estimate the concentration of the cell-consumed substance X (S102).

The determination unit 13c determines whether the concentration of the cell-consumed substance X estimated in S102 is equal to or higher than the threshold. When it is equal to or higher than the threshold (YES in S103), the second estimation unit 13d estimates the number of cells C by applying the concentration of the cell-consumed substance X estimated in S102 to the first relationship information stored in the memory unit 11 (S104).

On the other hand, when the estimated cellular metabolite concentration is lower than the threshold (NO in S103), the spectral analysis unit 13a measures the absorbance and the degree of scattering of the cell-produced substance Y contained in the culture medium in which the cells C are cultured (S105).

The first estimation unit 13b analyzes the data of the absorbance and the degree of scattering measured in S105 to estimate the concentration of the cell-produced substance Y (S106).

The second estimation unit 13d estimates the number of cells C by applying the concentration of cell-produced substance Y estimated in S106 to the second relationship information stored in the memory unit 11 (S107). In this way, the number of cells can be estimated at any stage of culture. Depending on the type of cells and the state of the culture, the concentrations of both cell-consumed substance and cell-produced substance may be below the thresholds. In such a case, the number of cells cannot be estimated, and thus the process is ended.

In the case where a target is such a cellular metabolite that the relationship between decrease in cell-consumed substance and increase in cell-produced substance is clear, such as glucose and lactate, the number of cells C may be estimated by applying the concentration of the cell-produced substance Y estimated in S106 to the second relationship information stored in the memory unit 11 in the second estimation unit 13d, without going through the step of S107.

### == Second example ==

Next, the following is a second example in which the cellular metabolite concentration is estimated, using an electrochemical system to obtain values corresponding to concentrations of the cellular metabolites contained in culture media, and analyzing the correlation between cellular metabolite concentration and the obtained electrical values.

### (Estimation of the number of cells)

The detailed description thereof is omitted since it is similar to the first example.

### (Device for measuring the number of cells)

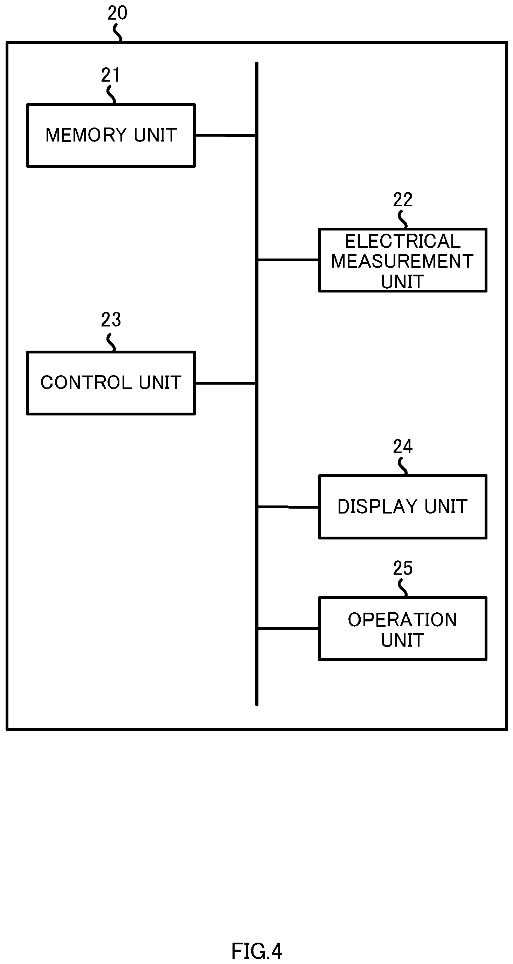

Referring to Fig. 4, a device 20 for estimating the number of cells according to the second example is described. The device 20 comprises a memory unit 21, an electrical measurement unit 22, a control unit 23, a display unit 24, and an operation unit 25.

### [Memory unit, display unit, and operation unit]

The configurations of the memory unit 21, the display unit 24, and the operation unit 25 are similar to those of the memory unit 11, the display unit 14, and the operation unit 15 in the first example, respectively. Thus, detailed descriptions thereof are omitted.

### [Electrical measurement unit]

The electrical measurement unit 22 measures electrical values associated with changes in specific cellular metabolites. The electrical measurement unit 22 can use the enzyme sensor described above. The electrical measurement unit 22 outputs the measured values to the control unit 23.

### [Control unit]

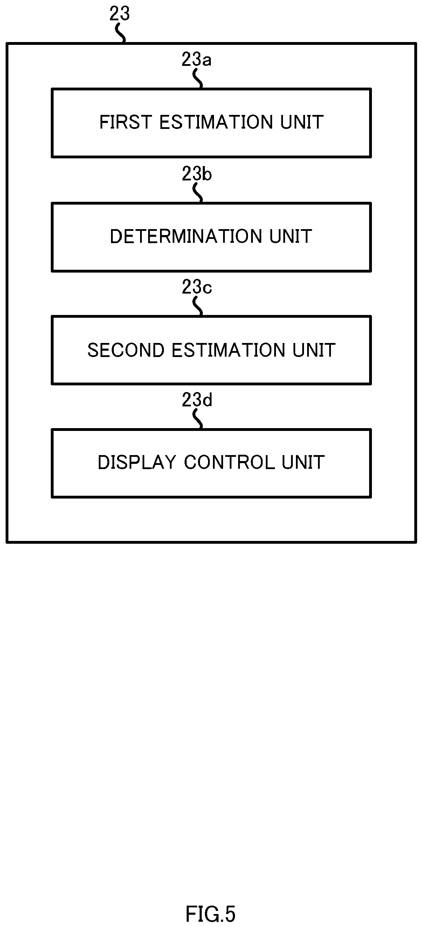

In an embodiment of the present disclosure, the control unit 23 functions as a first estimation unit 23a, a determination unit 23b, a second estimation unit 23c, and a display control unit 23d (see Fig. 5).

The first estimation unit 23a estimates the concentration of a cellular metabolite contained in the culture medium in which certain cells are cultured, based on electrical values measured using an electrochemical system. The method described above can be used to estimate the cellular metabolite concentration.

The determination unit 23b determines whether the estimated concentration of the cellular metabolite is equal to or higher than a threshold.

The second estimation unit 23c estimates the number of certain cells by applying the estimated concentration of the cellular metabolite to the relationship information stored in the memory unit 21, according to the determination result obtained by the determination unit 23b.

Here, a specific process according to an embodiment of the present disclosure is described. First, the first estimation unit 23a estimates the concentration of a first cellular metabolite. The determination unit 23b determines whether the estimated concentration of the first cellular metabolite is equal to or higher than the threshold.

When it is determined that the estimated concentration of the first cellular metabolite is equal to or higher than the threshold, the second estimation unit 23c estimates the number of certain cells by applying the estimated concentration of the first cellular metabolite to first relationship information stored in the memory unit 21.

On the other hand, when the estimated concentration of the first cellular metabolite is determined to be lower than the threshold, the first estimation unit 23a estimates the concentration of the second cellular metabolite contained in the culture medium. The determination unit 23b determines whether the estimated concentration of the second cellular metabolite is equal to or higher than a threshold.

When it is determined that the estimated concentration of the second cellular metabolite is equal to or higher than the threshold, the second estimation unit 23c estimates the number of certain cells by applying the estimated concentration of the second cellular metabolite to second relationship information stored in the memory unit 21.

The display control unit 23d performs various display controls in the device 20. The display control unit 23d displays the result of the estimation of the number of cells on the display unit 24, for example.

### (Method for estimating the number of cells)

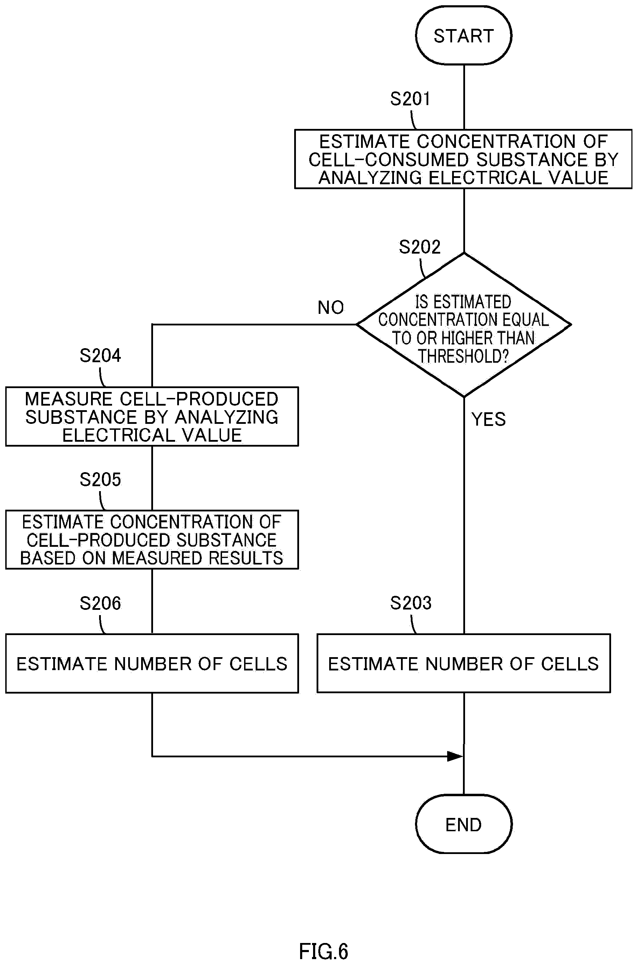

Next, referring to Fig. 6, described is a method for estimating the number of cells C using the device 20. An example is described assuming that cells C consume a cell-consumed substance X and produce a cell-produced substance Y. It is also assumed that the concentration of the cell-consumed substance X is estimated first. Furthermore, it is assumed that the first relationship information indicating the relationship between concentration of the cell-consumed substance X and the number of cells C, and the second relationship information indicating the relationship between concentration of the cell-produced substance and the number of cells C are previously obtained using a bioassay.

First, a culture medium in which cells C are cultured is placed in the device 20.

The electrical measurement unit 22 measures an electrical value associated with change in the cellular metabolite X and outputs it to the control unit 23.

The first estimation unit 23a analyzes the electrical value output from the electrical measurement unit 22 to estimate the concentration of the cell-consumed substance X (S201).

The determination unit 23b determines whether the concentration of the cell-consumed substance X estimated in S201 is equal to or higher than the threshold. When it is equal to or higher than the threshold (YES in S202), the second estimation unit 23c estimates the number of cells C by applying the concentration of the cell-consumed substance X estimated in S201 to the first relationship information stored in the memory unit 21 (S203).

On the other hand, when the estimated cellular metabolite concentration is lower than the threshold (NO in S202), the first estimation unit 23a analyzes the electrical value associated with change in the cellular metabolite Y output from the electrical measurement unit 22 to estimate the concentration of the cell-produced substance Y contained in the culture medium in which the cells C are cultured (S204).

The second estimation unit 23c estimates the number of cells C by applying the concentration of cell-produced substance Y estimated in S204 to the second relationship information stored in the memory unit 21 (S205). In this way, the number of cells can be estimated at any stage of culture. Depending on the type of cells and the state of the culture, the concentrations of both cell-consumed substance and cell-produced substance may be below the thresholds. In such a case, the number of cells cannot be estimated, and thus the process is ended.

### <Others>

The examples in Figs. 3 and 6 describe cases each in which two cellular metabolites are used, but the number of cellular metabolites is not limited thereto. In other words, the present disclosure is available for cases each in which three or more cellular metabolites are used. For example, in Fig. 3, when the concentration of the cell-produced substance Y estimated in S106 is lower than the threshold (NO in S107), the concentration of a cellular metabolite Z, which is different from the cell-consumed substance X and the cell-produced substance Y and which is contained in the culture medium in which cells C are cultured is estimated by using a method similar to the one described above. Then, the number of cells C is estimated by applying the estimated concentration of the cellular metabolite Z to the previously obtained relationship information indicating the relationship between concentration of cellular metabolite Z and the number of certain cells.

The cells to be subject to the method for estimating cells in the present disclosure can be used not only for cells involved in immunity of patients but also for other cells. In addition, the method can be used not only for human cells but also for cells of other animals and plants.

### == Examples ==

Although the present disclosure will be described in more detail with examples below, it should not be limited to these examples.

The following is an example in which the relationship between cellular metabolite concentration and the number of cells is obtained using a bioassay, and an example in which the cellular metabolite concentration is estimated using spectroscopy. In an example of the present disclosure, the cells to be cultured are T cells. The culture media were seeded with 5.0 × 10⁶ T cells. In an example of the present disclosure, the cellular metabolites are glucose, which is a cell-consumed substance, and lactate, which is a cell-produced substance, contained in the culture media. A detailed description will be given below.

### <Relationship between cellular metabolite concentration and the number of cells>

### (Experimental conditions and methods)

Glucose and lactate were individually reacted with the following reagents, and concentrations were measured through detecting luminescence signals. The number of cells was measured by a known method such as colony counting or the like. To confirm the reproducibility, the experiments were conducted with three samples prepared under the same conditions.

### (Reagents)

Glucose: Glucose-Glo Assay (Promega Corporation)
Lactate: Lactate-Glo Assay (Promega Corporation)

### (Luminescence detector)

SpectraMax iD3 (manufactured by Molecular Devices Japan Inc.)

### (Results)

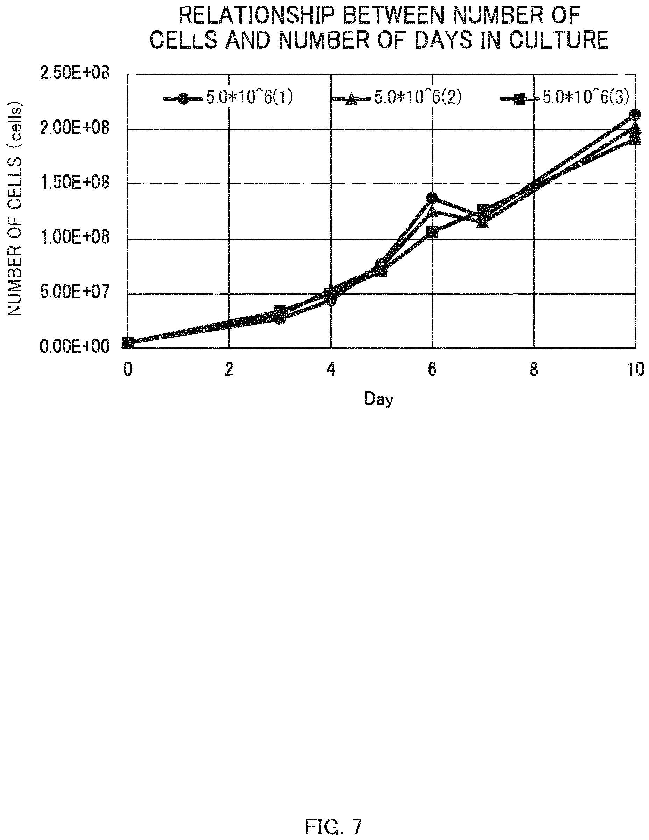

Fig. 7 is a graph illustrating the relationship between the number of T cells obtained using the bioassay and the number of days in culture. Specifically, as indicated by the markers in Fig. 7, measurements were taken immediately after seeding the T cells, 3, 4, 5, 6, 7, and 10 days after seeding.

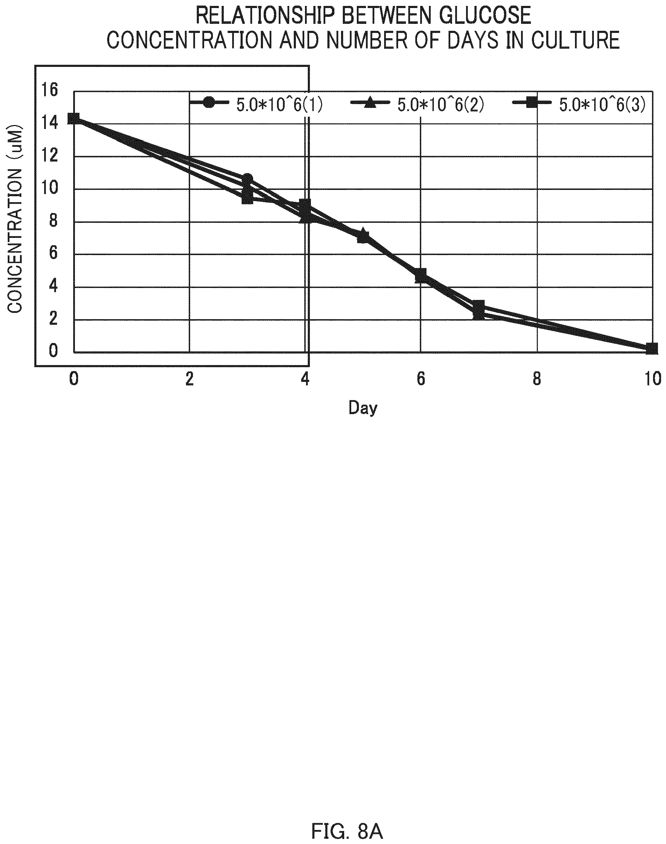

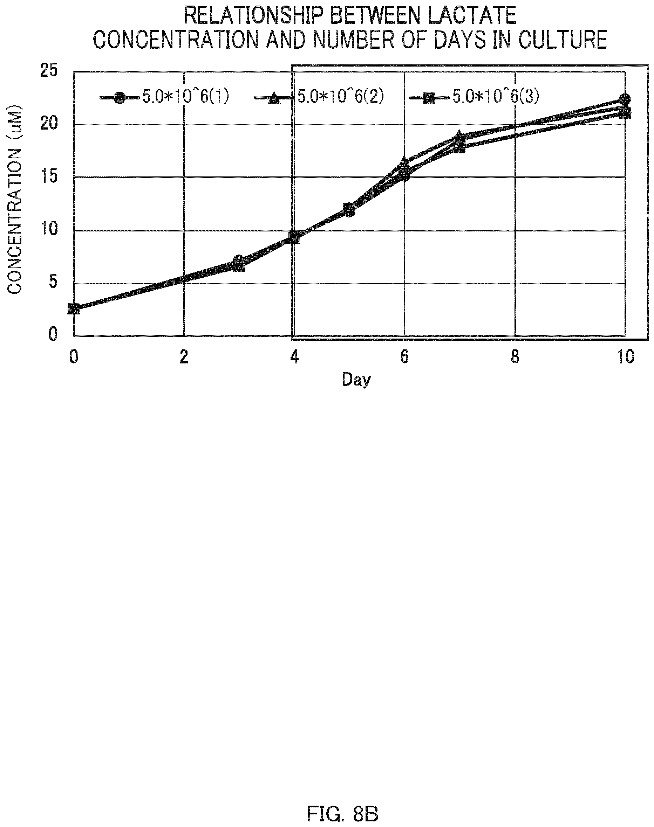

Fig. 8A is a graph illustrating the relationship between glucose concentration and the number of days in T-cell culture obtained using the bioassay. It can be seen that the concentration of glucose, which is a cell-consumed substance, decreases over time as the cells are cultured. Fig. 8B is a graph illustrating the relationship between lactate concentration and the number of days in T-cell culture obtained using the bioassay. It can be seen that the concentration of lactate, which is a cell-produced substance, increases over time as the cells are cultured. In the figure, "uM" stands for 10⁻⁶ mol/L.

It is possible to read, from Figs. 7, 8A, and 8B, relationship information indicating the relationship between concentrations of glucose and lactate, and the number of cells. For example, the number of T cells at 4 days after seeding of T cells is about 5 × 10⁷ when taking the average of three samples from Fig. 7. It can be read that the concentration of glucose at 4 days after seeding of T cells is about 8 × 10⁻⁶ mol/L from Fig. 8A, and the lactate concentration is about 10 × 10⁻⁶ mol/L from Fig. 8B. In other words, when the number of T cells is about 5 × 10⁷, the concentration of glucose is about 8 × 10⁻⁶ mol/L and the concentration of lactate is about 10 × 10⁻⁶ mol/L.

### <Estimation of cellular metabolite concentration>

In an example of the present disclosure, near-infrared spectroscopy was used as spectroscopy, and the concentration of T cells was estimated by measuring the absorbance of glucose and lactate.

### (Experimental conditions and methods)

### (Near-infrared wavelength)

The wavelength bandwidth of near-infrared light is 1069 to 2221 nm.

The absorption wavelength of glucose is 1600 ±30 nm.

The absorption wavelengths of lactate are 1587 to 1851 nm and 2083 to 2380 nm. For lactate, wavelengths greater than 2221 nm are outside the near-infrared range, and thus the wavelengths actually used are 1587-1851 nm and 2083-2221 nm.

### (Spectral measurement equipment)

A spectral measurement equipment (PHC Corporation) equipped with a C9914GB (Hamamatsu Photonics) was used as the spectral measurement part.

The absorbance was measured several times from the early stage to the terminal stage (10 days after seeding T cells) of cell culture.

The measured absorbance values were analyzed using multivariate analysis to estimate the concentration of T cells. Cross-validation was used as the method of multivariate analysis.

### (Results)

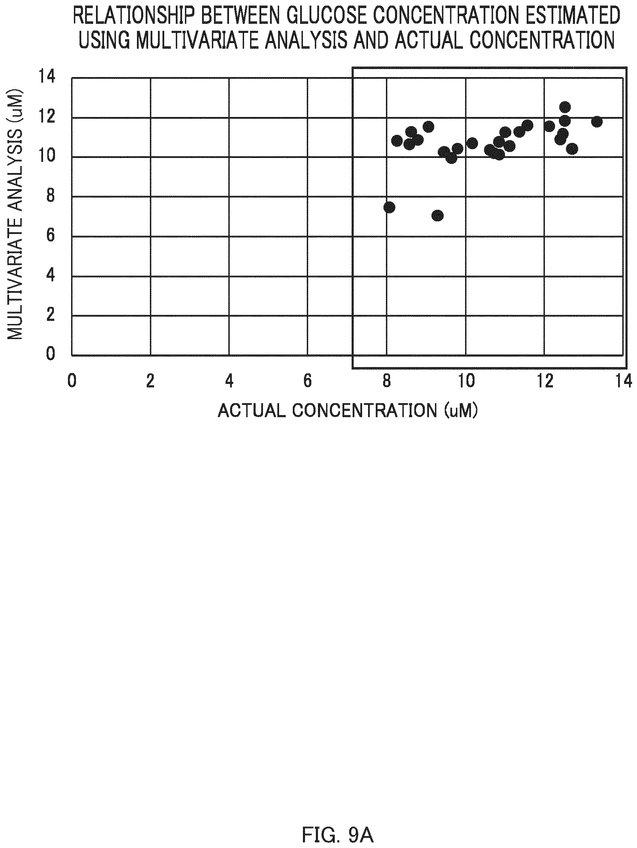

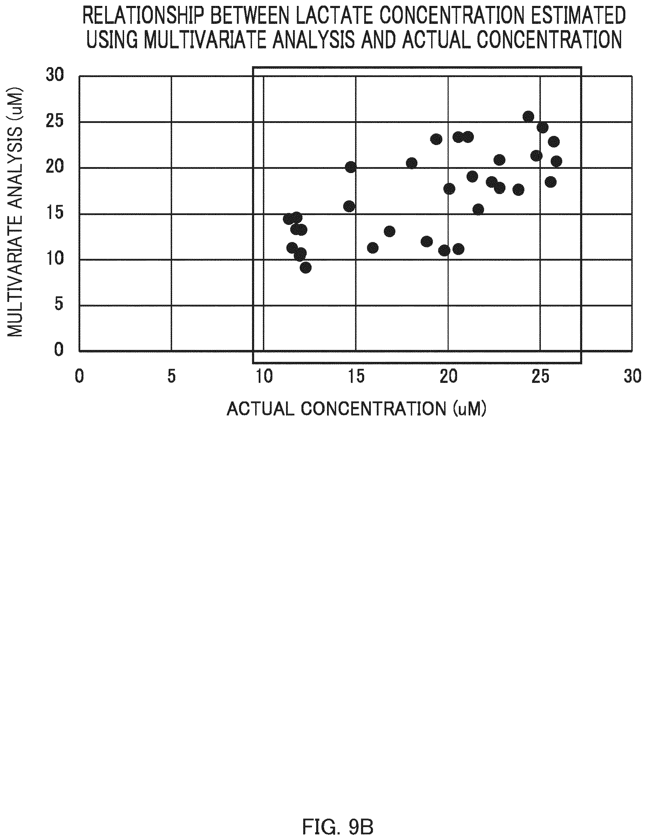

The results are illustrated in Figs. 9A and 9B. The horizontal axis in Fig. 9A is the glucose concentration obtained using the bioassay. The vertical axis in Fig. 9A is the glucose concentration estimated using multivariate analysis. The horizontal axis in Fig. 9B is the lactate concentration obtained using the bioassay. The vertical axis in Fig. 9B is the lactate concentration estimated using multivariate analysis. In other words, the results of the concentrations of glucose and lactate estimated using spectroscopy are the vertical axes of the dots in Figs. 9A and 9B.

When excluding the values of concentrations that are less than 8 × 10⁻⁶ mol/L for glucose and less than 10 × 10⁻⁶ mol/L for lactate from the concentrations estimated by spectroscopy, a correlation can be found between the concentrations estimated using multivariate analysis and those obtained using the bioassay. To confirm the correlation, multivariate analysis was used again. The multivariate analysis method used was the least squares method.

### <Summary>

As is clear from examples described above, the number of days in T-cell culture during which glucose concentrations are 8 × 10⁻⁶ mol/L or higher is 4 days from seeding of the T cells. In other words, the number of T cells can be estimated using glucose from when the T cells are seeded until when 4 days has elapsed since the seeding of the T cells. Lactate is a cellular metabolite to be produced by the consumption of glucose, and thus the concentration thereof increases as the number of days in T-cell culture increases. According to examples described above, the number of days in T-cell culture to be taken for the lactate concentrations to reach 10 × 10⁻⁶ mol/L or higher is 4 days or more from the seeding the T cells. In other words, the number of T cells can be estimated using lactate from when 4 days have elapsed since the seeding of the T cells to the terminal stage of cell culture (10 days in an example described above).

In this way, it can be seen that the number of T cells can be estimated in the entire range from the early stage to the terminal stage (10 days) of cell culture.

## Claims

1. A method for estimating the number of cells, the method comprising:
estimating (S102, S201) a concentration of a cell-consumed substance contained in a culture medium in which certain cells are cultured, using a spectroscopy or electrochemical system, the spectroscopy and electrochemical system being capable of obtaining a value corresponding to a concentration of a cellular metabolite contained in a culture medium;
estimating (S104, S203), when the estimated concentration of the cell-consumed substance is equal to or higher than a threshold, the number of the certain cells by applying the estimated concentration of the cell-consumed substance to previously obtained first relationship information indicating a relationship between a concentration of the cell-consumed substance and the number of the certain cells;
estimating (S106, S205), when the estimated concentration of the cell-consumed substance is lower than the threshold, a concentration of a cell-produced substance contained in the culture medium, using the spectroscopy or electrochemical system; and
estimating (S107, S206) the number of the certain cells by applying the estimated concentration of the cell-produced substance to previously obtained second relationship information indicating a relationship between a concentration of the cell-produced substance and the number of the certain cells.

2. A method for estimating the number of cells, the method comprising:
estimating a concentration of a cell-produced substance contained in a culture medium in which certain cells are cultured, using a spectroscopy or electrochemical system, the spectroscopy and electrochemical system being capable of obtaining a value corresponding to a concentration of a cellular metabolite contained in a culture medium;
estimating, when the estimated concentration of the cell-produced substance is equal to or higher than a threshold, the number of the certain cells by applying the estimated concentration of the cell-produced substance to previously obtained first relationship information indicating a relationship between a concentration of the cell-produced substance and the number of the certain cells;
estimating, when the estimated concentration of the cell-produced substance is lower than the threshold, a concentration of a cell-consumed substance contained in the culture medium, using the spectroscopy or electrochemical system; and
estimating the number of the certain cells by applying the estimated concentration of the cell-consumed substance to previously obtained second relationship information indicating a relationship between a concentration of the cell-consumed substance and the number of the certain cells.

3. The method according to claim 1 or 2, further comprising:
estimating, when both the estimated concentrations of the cell-consumed substance and cell-produced substance are lower than the thresholds respectively, a concentration of another cell-consumed substance or cell-produced substance contained in the culture medium, using the spectroscopy or electrochemical system; and
estimating the number of the certain cells by applying the estimated concentration of the other cell-consumed substance or cell-produced substance to previously obtained third relationship information indicating a relationship between a concentration of the other cell-consumed substance or cell-produced substance and the number of the certain cells.

4. The method according to any one of claims 1 to 3, wherein the first relationship information, the second relationship information, and the third relationship information are obtained using a bioassay.

5. The method according to any one of claims 1 to 4, wherein the concentrations are estimated using multivariate analysis.

6. The method according to any one of claims 1 to 5, wherein the spectroscopy is selected from a near-infrared spectroscopy, a visible spectroscopy, a fluorescence spectroscopy, and a Raman spectroscopy.

7. The method according to any one of claims 1 to 6, wherein the cell-consumed substance is glucose, the cell-produced substance is lactate, and the spectroscopy is a near-infrared spectroscopy.

8. The method according to claim 1 or 2, wherein
the thresholds each are lowest obtained by:
measuring, using a near-infrared spectroscopy, an absorbance of a culture medium with a given concentration of a cellular metabolite;
estimating, using multivariate analysis, a concentration of a cellular metabolite from the absorbance;
repeating the measuring and estimating, to accumulate data regarding a relationship between a measured concentration and an estimated concentration of a cellular metabolite;
deleting, using a cross-validation method, data of low concentrations from the measured data until a correlation coefficient between a measured concentration and an estimated concentration of a cellular metabolite becomes 0.7 or higher; and
setting each of the thresholds to a lowest concentration value of measured data obtained when the correlation coefficient is 0.7 or higher.

9. A device that estimates the number of cells, the device comprising:
a first estimation unit (13b, 23a) that estimates a concentration of a cell-consumed substance and a cell-produced substance contained in a culture medium in which certain cells are cultured, based on data measured using a spectroscopy or electrochemical system, the spectroscopy and electrochemical system being capable of obtaining a value corresponding to a concentration of a cellular metabolite contained in a culture medium;
a determination unit (13c, 23b) that determines whether the estimated concentration of the cell-consumed substance or cell-produced substance is equal to or higher than a threshold; and
a second estimation unit (13d, 23c) that estimates the number of certain cells, by applying the estimated concentration of the cell-consumed substance or cell-produced substance to previously obtained relationship information indicating a relationship between a concentration of the cell-consumed substance or cell-produced substance and the number of the certain cells, according to a determination result obtained by the determination unit (13c, 23b),
the second estimation unit (13d, 23c) being configured to, when the concentration of the cell-consumed substance estimated by the first estimation unit (13b, 23a) is determined to be equal to or higher than the threshold, estimate the number of the certain cells by applying the estimated concentration of the cell-consumed substance to previously obtained first relationship information indicating a relationship between a concentration of the cell-consumed substance and the number of the certain cells,
the second estimation unit (13d, 23c) being configured to, when the estimated concentration of the cell-produced substance is determined to be equal to or higher than the threshold, estimate the number of the certain cells by applying the estimated concentration of the cell-produced substance to previously obtained second relationship information indicating a relationship between a concentration of the cell-produced substance and the number of the certain cells.

## Patentansprüche

1. Verfahren zum Schätzen der Anzahl an Zellen, wobei das Verfahren umfasst:
Schätzen (S102, S201) einer Konzentration einer zellenverbrauchten Substanz, die in einem Kulturmedium enthalten ist, in dem bestimmte Zellen gezüchtet werden, unter Verwendung einer Spektroskopie oder eines elektrochemischen Systems, wobei die Spektroskopie und das elektrochemische System fähig sind, einen Wert zu erhalten, der einer Konzentration eines zellulären Stoffwechselprodukts entspricht, das in einem Kulturmedium enthalten ist;
Schätzen (S104, S203), wenn die geschätzte Konzentration der zellenverbrauchten Substanz gleich oder höher als ein Schwellenwert ist, der Anzahl der bestimmten Zellen, indem die geschätzte Konzentration der zellenverbrauchten Substanz auf vorher erhaltene erste Beziehungsinformation angewandt wird, die eine Beziehung zwischen einer Konzentration der zellenverbrauchten Substanz und der Anzahl der bestimmten Zellen anzeigt;
Schätzen (S106, S205), wenn die geschätzte Konzentration der zellenverbrauchten Substanz geringer als der Schwellenwert ist, einer Konzentration einer zellenerzeugten Substanz, die in dem Kulturmedium enthalten ist, unter Verwendung der Spektroskopie oder des elektrochemischen Systems; und
Schätzen (S107, S206) der Anzahl der bestimmten Zellen, indem die geschätzte Konzentration der zellenerzeugten Substanz auf vorher erhaltene zweite Beziehungsinformation angewandt wird, die eine Beziehung zwischen einer Konzentration der zellenerzeigten Substanz und der Anzahl der bestimmten Zellen anzeigt.

2. Verfahren zum Schätzen der Anzahl an Zellen, wobei das Verfahren umfasst:
Schätzen einer Konzentration einer zellenerzeugten Substanz, die in einem Kulturmedium enthalten ist, in dem bestimmte Zellen gezüchtet werden, unter Verwendung einer Spektroskopie oder eines elektrochemischen Systems, wobei die Spektroskopie und das elektrochemische System fähig sind, einen Wert zu erhalten, der einer Konzentration eines zellulären Stoffwechselprodukts entspricht, das in einem Kulturmedium enthalten ist;
Schätzen, wenn die geschätzte Konzentration der zellenerzeugten Substanz gleich oder höher als ein Schwellenwert ist, der Anzahl der bestimmten Zellen, indem die geschätzte Konzentration der zellenerzeugten Substanz auf vorher erhaltene erste Beziehungsinformation angewandt wird, die eine Beziehung zwischen einer Konzentration der zellenerzeugten Substanz und der Anzahl der bestimmten Zellen anzeigt;
Schätzen, wenn die geschätzte Konzentration der zellenerzeugten Substanz geringer als der Schwellenwert ist, einer Konzentration einer zellenverbrauchten Substanz, die in dem Kulturmedium enthalten ist, unter Verwendung der Spektroskopie oder des elektrochemischen Systems; und
Schätzen der Anzahl der bestimmten Zellen, indem die geschätzte Konzentration der zellenverbrauchten Substanz auf vorher erhaltene zweite Beziehungsinformation angewandt wird, die eine Beziehung zwischen einer Konzentration der zellenverbrauchten Substanz und der Anzahl der bestimmten Zellen anzeigt.

3. Verfahren nach Anspruch 1 oder 2, ferner umfassend:
Schätzen, wenn beide der geschätzten Konzentrationen der zellenverbrauchten Substanz und der zellenerzeugten Substanz jeweils geringer sind als die Schwellenwerte, einer Konzentration einer anderen zellenverbrauchten Substanz oder zellenerzeugten Substanz, die in dem Kulturmedium enthalten ist, unter Verwendung der Spektroskopie oder des elektrochemischen Systems; und
Schätzen der Anzahl der bestimmten Zellen, indem die geschätzte Konzentration der anderen zellenverbrauchen Substanz oder zellenerzeugten Substanz auf vorher erhaltene dritte Beziehungsinformation angewandt wird, die eine Beziehung zwischen einer Konzentration der anderen zellenverbrauchten Substanz oder zellenerzeugten Substanz und der Anzahl der bestimmten Zellen anzeigt.

4. Verfahren nach einem der Ansprüche 1 bis 3, wobei die erste Beziehungsinformation, die zweite Beziehungsinformation, und die dritte Beziehungsinformation unter Verwendung eines Bioassays erhalten werden.

5. Verfahren nach einem der Ansprüche 1 bis 4, wobei die Konzentrationen unter Verwendung multivariater Analyse geschätzt werden.

6. Verfahren nach einem der Ansprüche 1 bis 5, wobei die Spektroskopie aus einer Nahinfrarotspektroskopie, einer sichtbaren Spektroskopie, einer Fluoreszenzspektroskopie, und einer Raman-Spektroskopie gewählt ist.

7. Verfahren nach einem der Ansprüche 1 bis 6, wobei die zellenverbrauchte Substanz Glukose ist, die zellenerzeugte Substanz Laktat ist, und die Spektroskopie eine Nahinfrarotspektroskopie ist.

8. Verfahren nach Anspruch 1 oder 2, wobei
die Schwellenwerte jeweils am niedrigsten erhalten werden durch:
Messen, unter Verwendung einer Nahinfrarotspektroskopie, eines Absorptionsgrads eines Kulturmediums mit einer gegebenen Konzentration eines zellulären Stoffwechselprodukts;
Schätzen, unter Verwendung multivariater Analyse, einer Konzentration eines Stoffwechselprodukts aus dem Absorptionsgrad;
Wiederholen des Messens und Schätzens, um Daten zu sammeln in Bezug auf eine Beziehung zwischen einer gemessenen Konzentration und einer geschätzten Konzentration eines zellulären Stoffwechselprodukts;
Löschen, unter Verwendung eines Kreuzvalidierungsverfahrens, von Daten niedriger Konzentrationen von den gemessenen Daten, bis ein Korrelationskoeffizient zwischen einer gemessenen Konzentration und einer geschätzten Konzentration eines zellulären Stoffwechselprodukts 0.7 oder höher wird; und
Einstellen jedes der Schwellenwerte auf einen niedrigsten Konzentrationswert gemessener Daten, der erhalten wird, wenn der Korrelationskoeffizient 0.7 oder höher ist.

9. Vorrichtung, die die Anzahl an Zellen schätzt, wobei die Vorrichtung umfasst:
eine erste Schätzeinheit (13b, 23b), die eine Konzentration einer zellenverbrauchten Substanz und einer zellenerzeugten Substanz schätzt, die in einem Kulturmedium enthalten ist, in dem bestimmte Zellen gezüchtet werden, auf Grundlage von Daten, die unter Verwendung einer Spektroskopie oder eines elektrochemischen Systems gemessen werden, wobei die Spektroskopie und das elektrochemische System fähig sind, einen Wert zu erhalten, der einer Konzentration eines zellulären Stoffwechselprodukts entspricht, das in einem Kulturmedium enthalten ist;
eine Bestimmungseinheit (13c, 23b), die bestimmt, ob die geschätzte Konzentration der zellenverbrauchten Substanz oder zellenerzeugten Substanz gleich oder höher als ein Schwellenwert ist; und
eine zweite Schätzeinheit (13d, 23c), die die Anzahl bestimmter Zellen schätzt, indem die geschätzte Konzentration der zellenverbrauchten Substanz oder zellenerzeugten Substanz auf vorher erhaltene Beziehungsinformation angewandt wird, die eine Beziehung zwischen einer Konzentration der zellenverbrauchten Substanz oder zellenerzeugten Substanz und der Anzahl der bestimmten Zellen anzeigt, entsprechend einem Bestimmungsergebnis, das von der Bestimmungseinheit (13c, 23b) erhalten wird,
wobei die zweite Schätzeinheit (13d, 23c) eingerichtet ist, wenn bestimmt wird, dass die Konzentration der zellenverbrauchten Substanz, die von der ersten Schätzeinheit (13b, 23a) geschätzt wird, gleich oder höher als der Schwellenwert ist, die Anzahl der bestimmten Zellen zu schätzen, indem die geschätzte Konzentration der zellenverbrauchten Substanz auf vorher erhaltene erste Beziehungsinformation angewandt wird, die eine Beziehung zwischen einer Konzentration der zellenverbrauchten Substanz und der Anzahl der bestimmten Zellen anzeigt,
wobei die zweite Schätzeinheit (13d, 23c) eingerichtet ist, wenn bestimmt wird, dass die geschätzte Konzentration der zellenerzeugten Substanz gleich oder höher als der Schwellenwert ist, die Anzahl der bestimmten Zellen zu schätzen, indem die geschätzte Konzentration der zellenerzeugten Substanz auf vorher erhaltene zweite Beziehungsinformation angewandt wird, die eine Beziehung zwischen einer Konzentration der zellenerzeugten Substanz und der Anzahl der bestimmten Zellen anzeigt.

## Revendications

1. Procédé d'estimation d'un nombre de cellules, le procédé comprenant :
l'estimation (S102, S201) d'une concentration d'une substance consommée par les cellules contenue dans un milieu de culture dans lequel des cellules particulières sont cultivées, en utilisant un système spectroscopique ou électrochimique, le système spectroscopique et électrochimique étant capable d'obtenir une valeur correspondant à une concentration d'un métabolite cellulaire contenu dans un milieu de culture ;
l'estimation (S104, S203), lorsque la concentration estimée de la substance consommée par les cellules est supérieure ou égale à un seuil, du nombre des cellules particulières en appliquant la concentration estimée de la substance consommée par les cellules à des premières informations de relation obtenues précédemment indiquant une relation entre une concentration de la substance consommée par les cellules et le nombre des cellules particulières ;
l'estimation (S106, S205), lorsque la concentration estimée de la substance consommée par les cellules est inférieure au seuil, d'une concentration d'une substance produite par les cellules contenue dans le milieu de culture, en utilisant le système spectroscopique ou électrochimique ; et
l'estimation (S107, S206) du nombre des cellules particulières en appliquant la concentration estimée de la substance produite par les cellules à des deuxièmes informations de relation obtenues précédemment indiquant une relation entre une concentration de la substance produite par les cellules et le nombre des cellules particulières.

2. Procédé d'estimation d'un nombre de cellules, le procédé comprenant :
l'estimation d'une concentration d'une substance produite par les cellules et contenue dans un milieu de culture dans lequel des cellules particulières sont cultivées, en utilisant un système spectroscopique ou électrochimique, le système spectroscopique et électrochimique étant capable d'obtenir une valeur correspondant à une concentration d'un métabolite cellulaire contenu dans un milieu de culture ;
l'estimation, lorsque la concentration estimée de la substance produite par les cellules est supérieure ou égale à un seuil, du nombre des cellules particulières en appliquant la concentration estimée de la substance produite par les cellules à des premières informations de relation obtenues précédemment indiquant une relation entre une concentration de la substance produite par les cellules et le nombre des cellules particulières ;
l'estimation, lorsque la concentration estimée de la substance produite par les cellules est inférieure au seuil, d'une concentration d'une substance consommée par les cellules contenue dans le milieu de culture, en utilisant le système spectroscopique ou électrochimique ; et
l'estimation du nombre des cellules particulières en appliquant la concentration estimée de la substance consommée par les cellules à des deuxièmes informations de relation obtenues précédemment indiquant une relation entre une concentration de la substance consommée par les cellules et le nombre des cellules particulières.

3. Procédé selon la revendication 1 ou 2, comprenant en outre :
l'estimation, lorsque les deux concentrations estimées de la substance consommée par les cellules et de la substance produite par les cellules sont respectivement inférieures aux seuils, d'une concentration d'une autre substance consommée par les cellules ou d'une autre substance produite par les cellules contenue dans le milieu de culture, en utilisant le système spectroscopique ou électrochimique ; et
l'estimation du nombre des cellules particulières en appliquant la concentration estimée de l'autre substance consommée par les cellules ou de la substance produite par les cellules à des troisièmes informations de relation obtenues précédemment indiquant une relation entre une concentration de l'autre substance consommée par les cellules ou de la substance produite par les cellules et le nombre des cellules particulières.

4. Procédé selon l'une quelconque des revendications 1 à 3, dans lequel les premières informations de relation, les deuxièmes informations de relation et les troisièmes informations de relation sont obtenues en utilisant un essai biologique.

5. Procédé selon l'une quelconque des revendications 1 à 4, dans lequel les concentrations sont estimées en utilisant une analyse multivariée.

6. Procédé selon l'une quelconque des revendications 1 à 5, dans lequel la spectroscopie est choisie parmi une spectroscopie dans le proche infrarouge, une spectroscopie dans le visible, une spectroscopie par fluorescence et une spectroscopie Raman.

7. Procédé selon l'une quelconque des revendications 1 à 6, dans lequel la substance consommée par les cellules est le glucose, la substance produite par les cellules est le lactate, et la spectroscopie est une spectroscopie dans le proche infrarouge.

8. Procédé selon la revendication 1 ou 2, dans lequel les seuils sont chacun le plus bas obtenu en :
mesurant, à l'aide d'une spectroscopie dans le proche infrarouge, l'absorbance d'un milieu de culture présentant une concentration donnée d'un métabolite cellulaire ;
estimant, à l'aide d'une analyse multivariée, une concentration d'un métabolite cellulaire à partir de l'absorbance ;
répétant la mesure et l'estimation, pour accumuler des données concernant une relation entre une concentration mesurée et une concentration estimée d'un métabolite cellulaire ;
supprimant, par une méthode de validation croisée, les données de faibles concentrations des données mesurées jusqu'à ce qu'un coefficient de corrélation entre une concentration mesurée et une concentration estimée d'un métabolite cellulaire devienne supérieur ou égal à 0,7 ; et
fixant chacun des seuils à une valeur de concentration la plus faible parmi les données mesurées obtenues lorsque le coefficient de corrélation est supérieur ou égal à 0,7.

9. Dispositif qui estime le nombre de cellules, le dispositif comprenant :
une première unité d'estimation (13b, 23a) qui estime une concentration d'une substance consommée par les cellules et d'une substance produite par les cellules contenues dans un milieu de culture dans lequel certaines cellules sont cultivées, sur la base de données mesurées à l'aide d'un système spectroscopique ou électrochimique, le système spectroscopique et électrochimique étant capable d'obtenir une valeur correspondant à une concentration d'un métabolite cellulaire contenu dans un milieu de culture ;
une unité de détermination (13c, 23b) qui détermine si la concentration estimée de la substance consommée par les cellules ou de la substance produite par les cellules est supérieure ou égale à un seuil ; et
une seconde unité d'estimation (13d, 23c) qui estime le nombre des cellules particulières, en appliquant la concentration estimée de la substance consommée par les cellules ou de la substance produite par les cellules à des informations de relation obtenues précédemment indiquant une relation entre une concentration de la substance consommée par les cellules ou de la substance produite par les cellules et le nombre des cellules particulières, selon un résultat de détermination obtenu par l'unité de détermination (13c, 23b),
la seconde unité d'estimation (13d, 23c) étant configurée pour estimer, lorsque la concentration de la substance consommée par les cellules estimée par la première unité d'estimation (13b, 23a) est déterminée comme étant supérieure ou égale au seuil, le nombre des cellules particulières en appliquant la concentration estimée de la substance consommée par les cellules à des premières informations de relation obtenues précédemment indiquant une relation entre une concentration de la substance consommée par les cellules et le nombre des cellules particulières,
la seconde unité d'estimation (13d, 23c) étant configurée pour estimer, lorsque la concentration estimée de la substance produite par les cellules est déterminée comme étant égale ou supérieure au seuil, le nombre des cellules particulières en appliquant la concentration estimée de la substance produite par les cellules à des deuxièmes informations de relation obtenues précédemment indiquant une relation entre une concentration de la substance produite par les cellules et le nombre des cellules particulières.
